Europäisches Patentamt

European Patent Office

Office européen des brevets

(19)

(11) Publication number: **0 003 620**
**B1**

(12) **EUROPEAN PATENT SPECIFICATION**

(45) Date of publication of patent specification: **06.06.84**

(51) Int. Cl.³: **C 07 D 251/34**

(21) Application number: **79200036.6**

(22) Date of filing: **20.01.79**

(54) **Preparation of tris-(beta-hydroxypropyl) isocyanurate.**

(30) Priority: **23.01.78 NL 7800770**

(43) Date of publication of application:
**22.08.79 Bulletin 79/17**

(45) Publication of the grant of the patent:
**06.06.84 Bulletin 84/23**

(84) Designated Contracting States:
**BE CH DE FR GB IT**

(56) References cited:
**DE - A - 1 670 214**
**US - A - 3 313 812**
**US - A - 4 007 139**

**THE JOURNAL OF ORGANIC CHEMISTRY, vol. 28, 1963, Easton, Pennsylvania R.W. CUMMINS, "Reaction of Cyanuric Acid with Epoxides", pages 85-89.**
(73) Proprietor: **STAMICARBON B.V.**
**Postbus 10**
**NL-6160 MC Geleen (NL)**

(72) Inventor: **Frisch, Kurt C.**
**17986 Park Lane**
**Grosse Ile (Mich) 48138 (US)**
Inventor: **Tummers, Daniel Maria Joseph**
**Rubensstraat 21**
**NL-6165 TP Geleen (NL)**
Inventor: **Te Nijenhuis, Anne**
**Dr. Schaepmanstraat 52**
**NL-6444 XZ Brunssum (NL)**

(74) Representative: **Hatzmann, Marinus Jan et al,**
**OCTROOIBUREAU DSM P.O.Box 9**
**NL-6160 MA Geleen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a process of preparing tris-($\beta$-hydroxypropyl)isocyanurate, hereinafter referred to as THPIC. THPIC may be used as a polyol in the preparation of polyurethanes or polyesters.

It has already been proposed to convert cyanuric acid into tris-hydroxyalkyl isocyanurate by reaction of cyanuric acid with an alkylene oxide. As cyanuric acid is a solid, the reaction is effected in an inert solvent or vehicle, e.g., dimethyl formamide (see French Patent Specification 1,451,002), a lower alcohol or an ether (see German Patent Specification 1670214) or benzene. The reaction may be effected in the presence of an acid or basic catalyst, in most cases a strongly basic catalyst for example a tertiary amine or an alkali hydroxide. This known method of preparation has the drawback that the solvent or vehicle has to be removed upon completion of the reaction, and so have the traces of catalyst in case a catalyst has been added. The addition product is then obtained as a very viscous fluid in the case of tris-($\beta$-hydroxypropyl) isocyanurate or as a solid in the case of tris-(hydroxyethyl)isocyanurate, and when used as a polyol in the preparation of polyurethane, has to be dissolved again in the other reactants.

According to the invention tris-($\beta$-hydroxypropyl)isocyanurate is prepared by reaction of cyanuric acid with an at least stoichiometric amount of propylene oxide at a temperature in the range from 100° to 150°C at superatmospheric pressure without a catalyst in a suitable reaction medium, wherein the reaction medium used is a polyhydroxycompound that is liquid at the reaction temperature and in which tris-($\beta$-hydroxypropyl) isocyanurate is soluble or miscible, said polyhydroxycompound belonging to the group consisting of aliphatic and cycloaliphatic polyols with 2—15 carbon atoms, polyoxyalkylene glycols containing from 2 to 20 oxyalkylene units derived from ethylene oxide, propylene oxide or butylene oxide, adducts of a $C_{2-4}$ alkylene oxide with a $C_{2-12}$ aliphatic, cycloaliphatic or aromatic polyamine or monohydroxymonoamine or with a $C_{2-15}$ aliphatic or cycloaliphatic polyol, said adducts containing from 1 to 10 oxyalkylene units per amino- or hydroxylgroup.

It has been found in the process according to the invention that the cyanuric acid is converted into tris-($\beta$-hydroxypropyl)isocyanurate in a comparatively short time without appreciable formation of by-products. The reaction terminates upon addition of three hydroxypropyl groups to the isocyanurate ring without substantial propoxylation of the polyol used as a vehicle. It has furthermore been found that no appreciable further propoxylation of the tris-($\beta$-hydroxypropyl)isocyanurate occurs, even though excess propylene oxide is used.

The polyhydroxy compound may be any of such compounds that is liquid at the reaction temperature and with which tris-hydroxypropyl isocyanurate is miscible or is soluble in, and preferably which can be used as polyol in admixture with tris-($\beta$-hydroxypropyl)isocyanurate in the preparation of polyurethanes. Particular examples of such polyhydroxy compounds are diethylene glycol, triethylene glycol, dipropylene glycol; adducts of ethylene oxide and/or propylene oxide with ethylene diamine, diamino butane, isophorone diamine or toluene diamine. If so desired, a mixture of water and a diamino compound or a polyamine may be used as the reaction medium, when the amino hydrogen atoms are replaced in situ by a hydroxypropyl group. Other polyhydroxy compounds which may be used according to the invention are trihydroxy compounds for example trifunctional aliphatic alcohols e.g. glycerol and trimethylol propane, and adducts of an alkylene oxide with a polyamine or trihydroxy compound e.g. a glycerol-ethylene oxide adduct or a propyleneoxide-diethylenetriamine adduct.

The process according to the invention is effected at superatmospheric pressure, which has the effect of reducing the formation of coloured by-products. The pressure may with advantage be between 2 and 20 bars. In most instances a pressure is used which is between the vapour pressure of propylene oxide at the reaction pressure and 15 bars. At a lower pressure the reaction time is prolonged, and higher pressures do not provide any significant advantage.

The reaction may expeditiously be carried out at a temperature of between 100° and 150°C, and more expeditiously between 120° and 140°C.

In general, use may be made of a molar ratio of propylene oxide to cyanuric acid of 3:1 or higher, in most cases of between 3:1 and 5:1.

The amount of polyol used as solvent may be between 25 and 100% by weight, based on the amount of cyanuric acid, to achieve good results. Particularly good results are obtained with amounts of between 25 and 500% by weight. Use is preferably made of an amount of between 50 and 200% by weight.

The starting compound may be pure cyanuric acid or crude cyanuric acid containing for instance ammeline and ammelide.

The reaction according to the invention can be carried out by first introducing into a reaction vessel the propylene oxide and the polyol, and then adding cyanuric acid, or by adding propylene oxide to a dispersion of cyanuric acid in the polyol.

An advantage of the process according to the invention is that the hydroxyl groups once attached are not subjected to further propoxylation. Consequently the product has a constant composition, irrespective of any excess of propylene oxide present in the reaction.

The following Examples of the invention are provided.

Example I

87.2 grams of an adduct of propylene oxide with ethylene diamine (hydroxyl number 480) and 92 grams of cyanuric acid were introduced into a 1-litre autoclave. At a temperature of 130—135°C, 125 grams of propylene oxide were added with stirring over a period of 4 hours. A further 125 grams of propylene oxide and 92 grams of cyanuric acid were then added in separate portions with stirring. The propylene oxide was introduced at a pressure of 100 bars, the pressure during the reaction varying from 2 to 15 bars. The final product was a clear liquid consisting of 80% by weight of tris-($\beta$-hydroxy-propyl) isocyanurate and 20% by weight of the adduct of propylene oxide and ethylene diamine. Upon analysis it was found that free cyanuric acid was no longer present and that no detectable further propoxylation of the ethylene diamine/propylene oxide adduct used as the reaction medium had occurred.

Example II

In the manner set forth in Example I, cyanuric acid was propoxylated but using an adduct of ethylene oxide to toluene diamine (hydroxyl number 400), to form a mixture of 85% by weight of tris-($\beta$-hydroxypropyl) isocyanurate and 15% by weight of the adduct. The mixture had a hydroxyl number of 486, a viscosity at 71°C of 43.470 $Kg.m^{-1}.s^{-1}$ (43470 cP), and a specific gravity of 1.18 (20°C).

Example III

The procedure of Example I was repeated using 87 g of glycerol as the polyhydroxy compound, and a reaction time of one hour in the first stage and two hours in the second stage. No significant propoxylation of the gylcerol occurred and all cyanuric acid was converted.

**Claim**

A process of preparing tris-($\beta$-hydroxy-propyl)isocyanurate by reaction of at least a stoichiometric amount of propylene oxide with cyanuric acid at a temperature from 100°C to 150°C and at superatmospheric pressure in a suitable reaction medium without the addition of a catalyst, characterised in that the reaction is carried out using as the reaction medium a polyhydroxycompound that is liquid at the reaction temperature and in which tris-($\beta$-hydroxypropyl)isocyanurate is soluble or miscible, said polyhydroxycompound belonging to the group consisting of aliphatic and cycloaliphatic polyols with 2—15 carbon atoms, polyoxyalkylene glycols containing from 2 to 20 oxyalkylene units derived from ethylene oxide, propylene oxide or butylene oxide, adducts of a $C_{2-4}$ alkylene oxide with a $C_{2-12}$ aliphatic, cyclo-aliphatic or aromatic polyamine or mono-hydroxymonoamine or with a $C_{2-15}$ aliphatic or cycloaliphatic polyol, said adducts containing from 1 to 10 oxyalkylene units per amino- or hydroxylgroup.

**Revendication**

Procédé de préparation de tris-($\beta$-hydroxy-propyl)-isocyanurate par réaction d'au moins une quantité stoechiométrique de propylèneoxyde avec de l'acide cyanurique à une température allant de 100°C à 150°C et à une pression supérieure à la pression atmosphérique dans un milieu réactionnel approprié sans addition d'un catalyseur, caractérisé en ce que la réaction est conduite en utilisant comme milieu réactionnel un composé polyhydroxy qui est liquide à la température de la réaction et dans lequel le tris-($\beta$-hydroxypropyl)iso-cyanurate est soluble ou avec lequel il est, miscible, ledit composé polyhydroxy appartenant au groupe constitué par les polyols aliphatiques et cycloaliphatiques en $C_2$ à $C_{15}$, les polyoxyalcoylèneglycols contenant de 2 à 20 unités oxyalcoylène dérivées de l'éthylène-oxyde, du propylèneoxyde ou du butylène-oxyde, les produits d'addition d'un alcoylène-oxyde en $C_2$ à $C_4$ avec une polyamine en $C_2$ à $C_{12}$ aliphatique, cycloaliphatique ou aromatique ou une monohydroxymonoamine ou avec un polyol en $C_2$ à $C_{15}$ aliphatique ou cycloaliphatique, lesdits produits d'addition contenant de 1 à 10 unités oxyalcoylène par groupe amino ou hydroxyle.

**Patentanspruch**

Verfahren zur Herstellung von Tris-(beta-Hydroxypropyl) Isocyanurat durch Reaktion von mindestens einer stöchiometrischen Menge Propylenoxid mit Cyanursäure bei einer Temperatur zwischen 100°C und 150°C und einem überatmosphärischen Druck in einem geeigneten Reaktionsmittel ohne Zusatz eines Katalysators, dadurch gekennzeichnet, dass die Reaktion ausgeführt wird unter Verwendung einer Polyhydroxylverbindung als Reaktionsmittel, welche bei der Reaktionstemperatur flüssig ist und in bzw. mit der Tris-(beta-Hydroxypropyl) Isocyanurat löslich oder mischbar ist, wobei die genannte Polyhydroxylverbindung zu der Gruppe gehört, die besteht aus aliphatischen und cykloaliphatischen Polyolen mit 2—15 Kohlenstoffatomen, Polyoxyalkylenglykolen enthaltend 2 bis 20 Oxyalkylen-Ein-heiten, abgeleitet von Ethylenoxid, Propylen-oxid oder Butylenoxid, Addukte eines $C_{2-4}$ Alkylenoxids mit einem $C_{2-12}$ aliphatischen, cykloaliphatischen oder aromatischen Polyamin oder Monohydroxymonoamin oder mit einem $C_{2-15}$ aliphatischen oder cykloalipha-tischen Polyol, wobei die genannten Addukte zwischen 1 und 10 Oxyalkylen-Einheiten je Amino- oder Hydroxylgruppe enthalten.